# FASCICULE DE BREVET EUROPEEN

(11) **EP 2 109 609 B1**
(45) Date de publication et mention de la délivrance du brevet: **12.06.2013**
(21) Numéro de dépôt: 08761775.9
(22) Date de dépôt: 17.01.2008
(51) Int. Cl.: C07D 453/02

(54) **NOUVEAU DÉRIVE DE LA QUINUCLIDINE UTILE DANS LA PRÉPARATION DE LA MEQUITAZINE**
NEUARTIGE CHINUCLIDINDERIVATE ZUR HERSTELLUNG VON MEQUITAZIN
NOVEL QUINUCLIDINE DERIVATIVE USEFUL IN THE PREPARATION OF MEQUITAZINE

(30) Priorité: 18.01.2007 FR 0752742
(43) Date de publication de la demande: 21.10.2009
(73) Titulaire: Pierre Fabre Medicament, 92100 Boulogne-Billancourt (FR)
(72) Inventeur: MIOSKOWSKI, Charles, F-67200 Strasbourg (FR); GONNOT, Vanessa, F-44240 Suce En Erdre (FR); BAATI, Rachid, F-67460 Souffel Weyersheim (FR); NICOLAS, Marc, F-81600 Gaillac (FR)
(74) Mandataire: Regimbeau
(86) Numéro de dépôt international: PCT/FR2008/000056
(87) Numéro de publication internationale: WO 2008/107545

(56) Documents cités:
- EP-A- 0 089 860
- EP-A2- 1 074 552
- WO-A-99/29692
- FR-A1- 2 777 278
- DATABASE WPI DERWENT PUBLICATIONS LTD., LONDON, GB; 1992, XP002449126 Database accession no. 1992-253542 [31]

## Description

La présente invention est relative à un nouveau procédé de préparation de la méquitazine : 10-[1-aza-bicyclo (2,2,2)oct-3-yl]-méthyl-10H-phénothiazine.

La méquitazine est un composé utilisé comme médicament antihistaminique pour le traitement des allergies. Elle est décrite dans le brevet FR-0 064 605 par sa structure chimique et son activité thérapeutique. Son isomère lévogyre est décrit dans le brevet FR-2 522 660 EP-089 860. Le procédé de préparation de la méquitazine et de son isomère lévogyre est également décrit dans ces brevets. Ce dernier présente un certain nombre d'inconvénients.

La synthèse se fait, avec un faible rendement, par alkylation du 3-(chlorométhyl)quinuclidine avec le sel de sodium ou de potassium de la phénothiazine dans un solvant inerte à haut point d'ébullition comme le xylène, éventuellement additionné d'un co-solvant comme le HMPT, toxique. Un autre inconvénient est lié à l'utilisation de l'amidure de sodium qui provoque un dégagement important d'ammoniac gazeux.

La réaction n'est pas propre, un grand nombre de sous-produits est engendré. En particulier, le produit d'élimination d'HCl de la 3-(chlorométhyl)quinuclidine en 3-(méthylène) quinuclidine, est formé dans ces conditions de réaction, ce qui engendre une perte de rendement.

Des produits secondaires sont également formés au moment de la création de l'anion de la phénothiazine, que ce soit avec l'amidure de sodium, l'hydrure de sodium, le tertiobutylate de potassium, etc. D'intenses colorations du milieu réactionnel sont le signe de la délocalisation de l'anion sur les carbones des homocycles benzéniques qui conduit à la formation de sous-produits.

La présence de nombreuses impuretés de la réaction rend nécessaire des traitements chromatographiques, pour isoler la méquitazine pure. Enfin, les rendements ainsi obtenus sont faibles.

D'autres brevets ont trait à la préparation de la méquitazine. Par exemple, JP-KOKAI 04 169 583 (Chem. Abstr. : 118 : 6983) et JP-KOKAI 05 140 157 (Chem. Abstr. :120 :8602) (Utilisation de l'époxyde spiro de la quinuclidine sur le dérivé potassique de la phénothiazine), et EP 1074552.

Bien que ces brevets soient des améliorations des préparations antérieures, le problème de l'obtention de la méquitazine avec un bon rendement n'est pas résolu.

Le document FR2777278 décrit la préparation de la 3-(hydroxyméthyl)quinuclidine pour servir d'intermédiaire de synthèse à la préparation de la méquitazine. Selon ce document, les produits de départ peuvent être l'oxyde de 3-(méthylène)quinuclidine ou la 3-quinuclidinone qui sont transformés en 3-(hydroxyméthyl)quinuclidine via le 3-méthoxyméthyl-3-quinuclidinol.

Le document W09929692 décrit un procédé de synthèse de la méquitazine et de son isomère lévogyre. Le procédé décrit consiste à faire réagir le dérivé lithié de la phénothiazine avec le mésylate de la 3-(hydrométhyl)quinuclidine ; ce dernier étant aussi décrit comme nouvel intermédiaire pour la synthèse de la méquitazine.

La présente invention se propose de fournir un nouveau composé, en tant qu'intermédiaire de synthèse de médicaments dérivés de la quinuclidine et en particulier de la méquitazine.

La présente invention propose un procédé de préparation de ce nouvel intermédiaire ainsi qu'une nouvelle voie de synthèse de la méquitazine à partir de ce nouvel intermédiaire de synthèse.

A cet effet, la présente invention concerne le 1-aza-bicyclo[2.2.2]oct-2-en-3-ylméthyl acétate (5) de formule suivante :

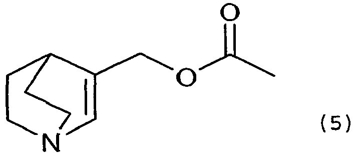

Ce composé n'a jamais été décrit dans l'art antérieur et peut servir d'intermédiaire pour la synthèse de la méquitazine via une amination allylique avec la phénothiazine.

Ce composé (5) peut être obtenu à partir de l'alcool allylique de la quinuclidine, ou 1-aza-bicyclo[2.2.2]oct-2-èn-3-ylméthanol, (3) de formule suivante :

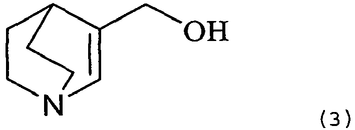

Cet alcool allylique (3) a été précédemment synthétisé par réduction de l'ester méthylique correspondant, lui-même obtenu par déshydratation puis hydrolyse du cyanoalcool issu de la quinuclidinone (US 4937239, JP 60258187, US 4467095), ce qui représente 4 étapes jusqu'à l'ester.

La présente invention décrit deux voies de synthèse plus compétitives pour ce composé (3), comprenant chacune seulement deux étapes.

Il peut se préparer selon deux voies possibles à partir de la quinuclidinone (1) :
Première voie : 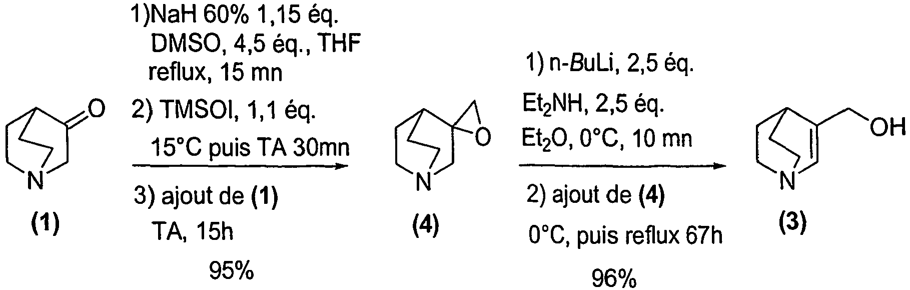
Seconde voie :

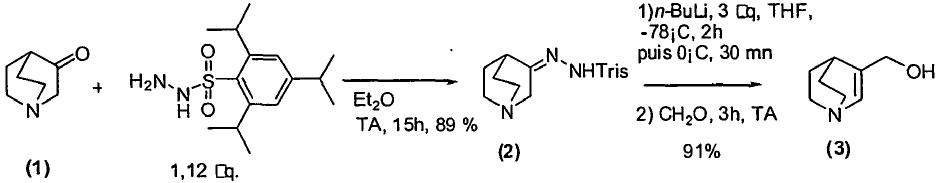

La première voie consiste à préparer la 3-époxyquinuclidine (4) :

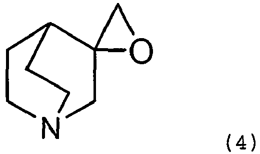

A cette fin, de l'hydrure de sodium à 60 % dans l'huile(1,14 g, 28,5 mmoles, 1,15 éq.) est mis en suspension dans le tétrahydrofurane anhydre (8 mL) puis le mélange est placé au reflux. Du diméthylsulfoxyde est ajouté et on observe un violent dégagement d'hydrogène. L'agitation est poursuivie 15 min puis le mélange est placé à 15°C et le triméthylsulfoxonium iodide (6 g, 27,26 mmoles, 1,1 éq.) est ajouté, et l'agitation poursuivie 30 min à température ambiante. Le mélange réactionnel devient de plus en plus épais. La 3-quinuclidinone (1) (3,1 g, 24,8 mmoles, 1 éq.) dissoute dans du tétrahydrofurane (3 mL) est ajoutée. On observe un dégagement de chaleur, et l'agitation est poursuivie pendant 15 h. De l'eau est ajoutée (100 mL), et le mélange est extrait 6 fois par du chloroforme. La phase organique est lavée plusieurs fois à l'eau, puis est séchée sur Na₂SO₄, filtrée et évaporée pour donner une huile jaune (2,3 g, 67%).

L'époxyde (4) ainsi obtenu subit une déprotonation en b pour donner le composé (3) selon le mode opératoire suivant : du *n*-Butyllithium (1,2 mL, 1,96 mmoles, 2,5 éq.) est ajouté à 0°C à une solution de diéthylamine (0,20 mL, 1,96 mmoles, 2,5 éq.) dans de l'éther diéthylique anhydre (5 mL). Le mélange est agité pendant 10 min puis l'époxyde (4) (109 mg, 0,79 mmoles, 1 éq.) dissout dans de l'éther diéthylique (1 mL) est ajouté à 0°C. Le mélange est agité au reflux pendant 67 h. Une solution aqueuse de carbonate de potassium est ajoutée, puis le mélange est extrait par du dichlorométhane et de l'éther. Les phases organiques sont séchées sur Na₂SO₄, filtrées et évaporées pour donner une huile jaune (105 mg, 96%).

Selon une autre voie alternative, le composé (3) peut être obtenu par une réaction de Shapiro sur l'hydrazone (2) :

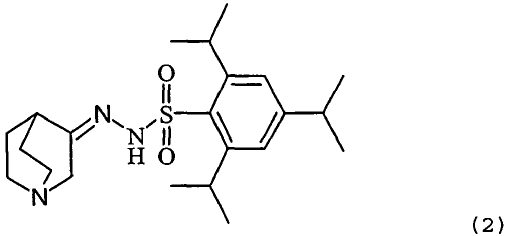

Cette hydrazone (2) peut être obtenue, toujours à partir de la 3-quinuclidinone, selon la procédure suivante :
La triisopropyl phényl hydrazine (20,3 g, 68 mmoles, 1,12 éq.) est dissoute dans du diéthyl éther anhydre (250 mL). Une solution de 3-quinuclidinone (1) (7,54 g, 60,3 mmoles, 1 éq.) dans du diéthyléther (100 mL) est canulée à température ambiante et le mélange est vivement agité pendant 15 h. Le précipité blanc est filtré puis lavé trois fois avec un minimum de diéthyléther, puis séché sous vide (21,84 g, 89%).

La réaction de Shapiro sur l'hydrazone (2) mène à l'alcool allylique (3) selon un mode opératoire tel que décrit ci-dessous :

L'hydrazone (2) (10,26 g, 25,3 mmoles, 1 éq.) est dissoute dans du tétrahydrofurane anhydre (50 mL) et placée à -78°C. Du *n*-Butyllithium (47,5 mL, 76 mmoles, 3 éq.) est ajouté pendant 30 min. Le mélange devient rouge et est agité pendant 2 h, puis il est placé à 0°C. De l'azote se dégage alors pendant environ 15 min, et le mélange devient jaune orangé. Le paraformaldéhyde (2,28 g, 76 mmoles, 3 éq.) est ajouté à 0°C puis le mélange est agité à température ambiante pendant 3 h et devient jaune limpide. De l'eau et du carbonate de potassium sont ajoutés et le mélange extrait par de l'éther diéthylique. Les phases organiques sont séchées sur Na₂SO₄, filtrées et évaporées et le résidu est purifié sur silice (dichlorométhane/ méthanol/triéthylamine : 9/1/0,1) pour donner une huile jaune (6,4 g, 91 %).

Comme indiqué plus avant, le composé (3) peut servir pour la synthèse de méquitazine, via une substitution allylique à partir du dérivé acétate de (3), suivi d'une hydrogénation catalytique de la déhydroméquitazine (6).

Ainsi, le composé (5) peut être obtenu à partir de l'alcool allylique (3): 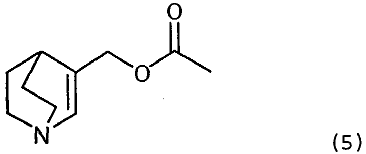 selon la procédure suivante :
l'alcool allylique (3) (126 mg, 0,9 mmoles, 1 éq.) est placé dans du dichlorométhane (4 mL) à 0°C. De l'anhydride acétique (0,1 mL, 1,1 mmoles, 1,2 éq.) est ajouté puis le mélange est agité pendant 3 h. De l'eau est ajoutée et les phases sont séparées. La phase aqueuse est basifiée avec une solution aqueuse à 10% de carbonate de potassium puis extraite avec du dichlorométhane. Les phases organiques sont rassemblées, séchées sur Na₂SO₄, filtrées et évaporées pour donner une huile orange (137,6 mg, 84 %).

Le composé (5) obtenu est complexé avec du Palladium (0) et mis en réaction avec la phénothiazine pour obtenir la déhydroméquitazine (6): 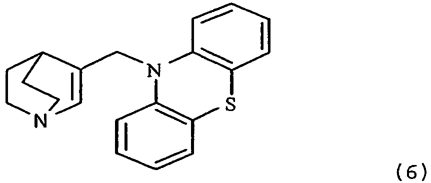 selon la procédure suivante :
la phénothiazine (6,6 g, 33 mmoles, 1,5 éq.) est placée dans du tétrahydrofurane (50 mL) à température ambiante, puis de l'hydrure de sodium (880 mg, 36,4 mmoles, 1,65 éq.) est ajouté. L'agitation est poursuivie pendant 20 min à température ambiante et l'anion est de couleur vert foncé.

### Préparation du complexe:

Parallèlement, le tétrakis(triphénylphosphine) palladium (1g, 0,66 mmoles, 4%) est ajouté sur l'acétate (5) (4 g, 22 mmoles, 1 éq.) en solution dans du tétrahydrofurane (50 mL), et le mélange est agité à température ambiante pendant 10/15 minutes (complexe rouge).

Le complexe précédemment formé est canulé sur l'anion, et le mélange est agité pendant 3h. Une solution aqueuse saturée de carbonate de potassium est ajoutée et le mélange est extrait par du dichlorométhane. Les phases organiques sont séchées sur Na₂SO₄, filtrées puis évaporées. L'huile verte obtenue est purifiée sur silice dichlorométhane/ méthanol/ triéthylamine : 96/4/0,5) et on obtient un solide beige (4 g, 58 %).

La déhydroméquitazine (6) subit ensuite une hydrogénation catalytique selon la procédure suivante :
La déhydroméquitazine (6) (51 mg, 0,16 mmoles, 1 éq.) est dissoute dans du méthanol anhydre dégazé (2 mL) et du toluène anhydre dégazé (1 mL) puis le mélange est canulé dans un réacteur préalablement purgé 3 fois vide /Argon. Le catalyseur Pd/C (5 mg, 10 % massique) est ajouté puis le réacteur est mis sous H₂, purgé 3 fois à 5 bars d'H₂ puis mis sous 100 bars et agité pendant 24 h. Du charbon actif est ajouté, et le mélange est filtré sur Celite puis évaporé pour donner une huile jaune (47 mg, 91 %).

On obtient ainsi la méquitazine (7) sous forme racémique :

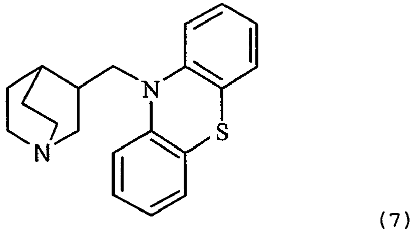

Une vue générale des voies d'obtention tant de la méquitazine (7), de l'alcool allylique (3) que du composé (5) objet de l'invention est figuré ci-dessous : 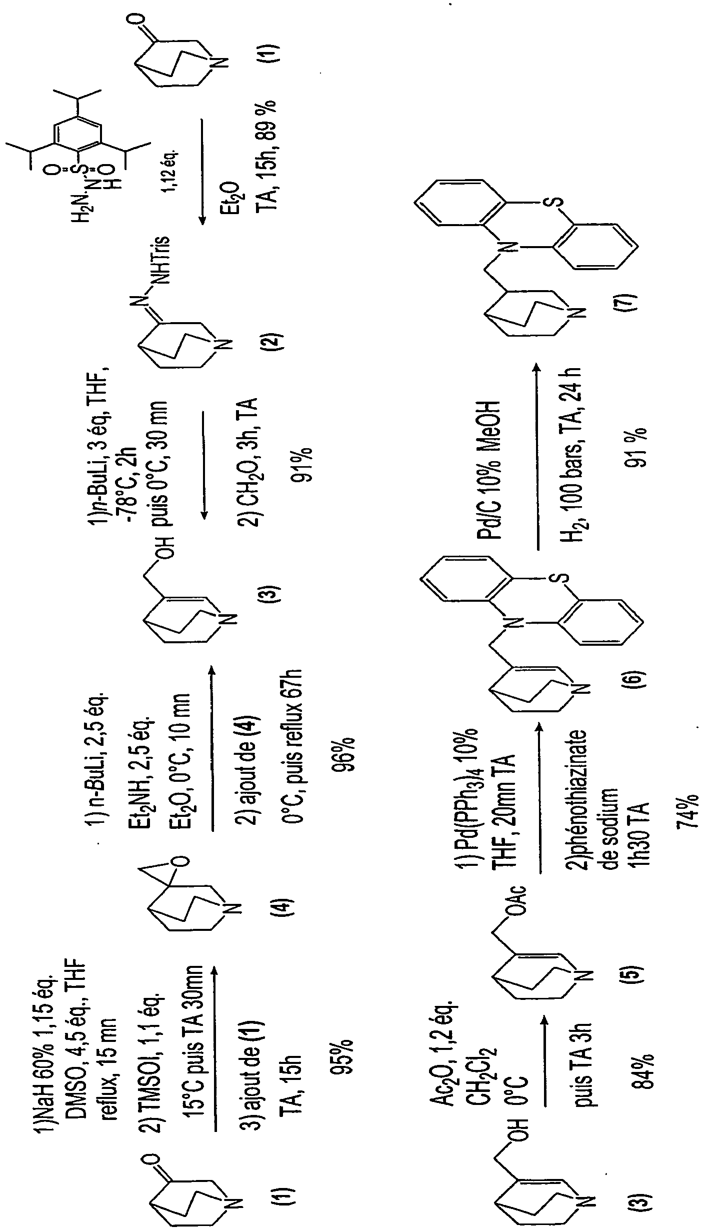 Ces procédés de préparation ne font intervenir qu'un nombre limité de stades de synthèse, présentent en outre de bons rendements et sont industrialisables.

L'invention concerne donc également un procédé de préparation de la méquitazine en utilisant le 1-aza-bicyclo[2.2.2]oct-2-en-3-ylméthyl acétate (5).

La méquitazine obtenue selon le procédé décrit ici, est sous forme de mélange racémique. Si l'on désire obtenir l'isomère lévogyre tel que décrit dans FR 2522 660 il conviendra de procéder à une hydrogénation catalytique énantiosélective de la déhydroméquitazine. Cette hydrogénation catalytique énantiosélective peut être réalisée grâce à l'utilisation d'un catalyseur de type ML*, dans lequel M représente un métal de transition, tel le Ruthénium ou le Rhodium, et L un ligand chiral, par exemple, le (R,R)Rh(cod)(DIPAMP)BF₄.

## Revendications

1. Le 1-aza-bicyclo[2.2.2]oct-2-èn-3-ylméthyl acétate de formule (5)

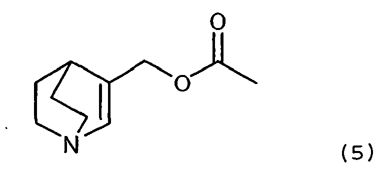

2. Utilisation du 1-aza-bicyclo[2.2.2]oct-2-èn-3-ylméthyl acétate de formule (5) selon la revendication 1, en tant qu'intermédiaire de synthèse dans la fabrication de médicaments dérivés de la quinuclidine.

3. Utilisation selon la revendication 2, **caractérisée en ce que** le dérivé de quinuclidine est la méquitazine de formule (7) aussi bien sous forme d'un mélange racémique que de mélanges d'isomères optiques en toutes proportions, et en particulier sous forme d'isomère lévogyre pur ou dextrogyre pur.

4. Procédé de fabrication de la méquitazine de formule (7) 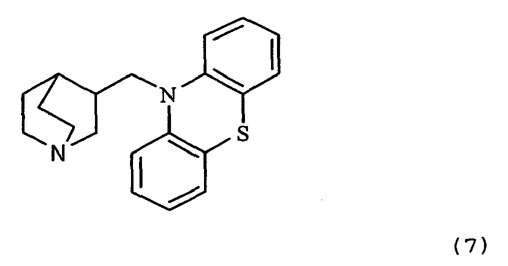 impliquant la séquence d'étapes réactionnelles suivantes :
(i) complexation au palladium de l'acétate de formule (5) selon la revendication 1 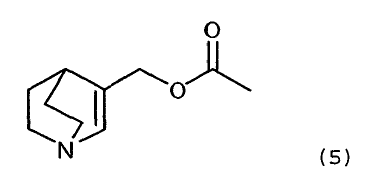
(ii) suivie d'une réaction d'amination allylique avec la phénothiazine conduisant à la dihydroméquitazine de formule (6) 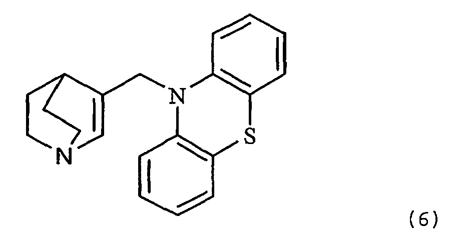
(iii) cette dernière étant soumise à une hydrogénation catalytique conduisant à la méquitazine de formule (7).

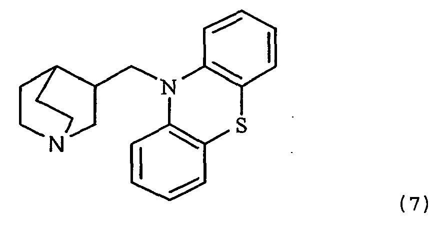

5. Procédé selon la revendication 4, **caractérisé en ce que** l'hydrogénation catalytique est conduite dans des conditions énantiosélectives.

6. Procédé pour la préparation de l'acétate de formule (5) selon la revendication 1, **caractérisé en ce qu'**il est obtenu par substitution allylique de l'alcool allylique de la quinuclidine de formule (3)

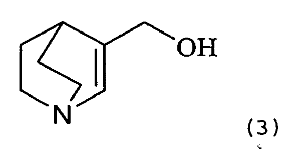

## Patentansprüche

1. 1-Azabicyclo[2.2.2]oct-2-en-3-ylmethylacetat der Formel (5)

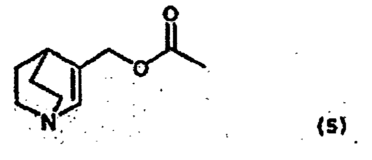

2. Verwendung von 1-Azabicyclo[2.2.2]oct-2-en-3-ylmethylacetat der Formel (5) nach Anspruch 1 als Synthesezwischenprodukt bei der Herstellung von von Chinuclidin abgeleiteten Arzneimitteln.

3. Verwendung nach Anspruch 2, **dadurch gekennzeichnet, dass** das Chinuclidin-Derivat Mequitazin der Formel (7) sowohl in Form einer racemischen Mischung als auch von Mischungen von optischen Isomeren in jeglichen Verhältnissen und insbesondere in Form von reinem linksdrehendem oder reinem rechtsdrehendem Isomer ist.

4. Verfahren zur Herstellung von Mequitazin der Formel (7) 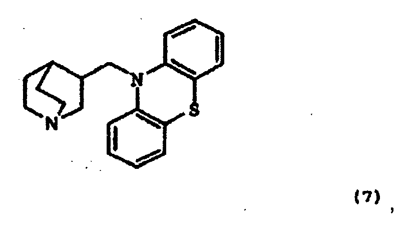 welches die Abfolge von folgenden Reaktionsschritten umfasst:
(i) Komplexierung des Acetats der Formel (5) nach Anspruch 1 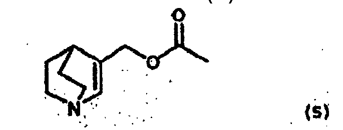 mit Palladium,
(ii) gefolgt von einer allylischen Aminierungsreaktion mit Phenothiazin, welche zu dem Dihydromequitazin der Formel (6) 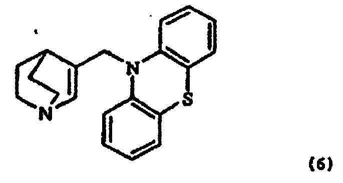 führt,
(iii) wobei dieses Letztere einer katalytischen Hydrierung unterworfen wird, welche zu Mequitazin der Formel (7) 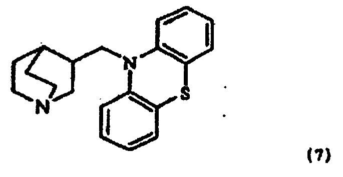 führt.

5. Verfahren nach Anspruch 4, **dadurch gekennzeichnet, dass** die katalytische Hydrierung unter enantioselektiven Bedingungen ausgeführt wird.

6. Verfahren zur Herstellung des Acetats der Formel (5) nach Anspruch 1, **dadurch gekennzeichnet, dass** es durch allylische Substitution des allylischen Alkohols von Chinuclidin der Formel (3) 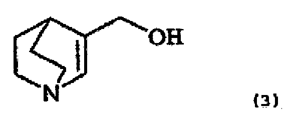 erhalten wird.

## Claims

1. 1-aza-bicyclo[2.2.2]oct-2-en-3-ylmethyl acetate of formula (5)

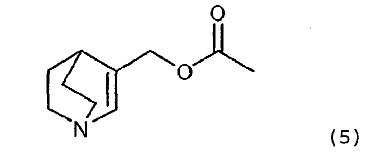

2. Use of 1-aza-bicyclo[2.2.2]oct-2-en-3-ylmethyl acetate of formula (5) according to claim 1 as a synthesis intermediate in the manufacture of quinuclidine derived medications.

3. Use according to claim 2, **characterised in that** the quinuclidine derivative is mequitazine of formula (7) in the form of both a racemic mixture and optic isomer mixtures in all proportions, particularly in the form of a pure levorotatory isomer or pure dextrorotatory isomer.

4. Process for the manufacture of mequitazine of formula (7) 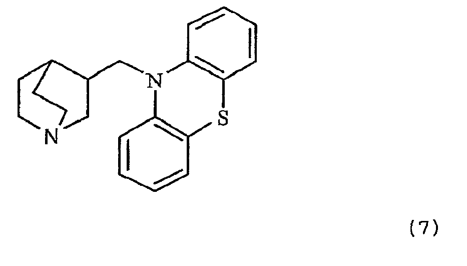 involving the following reaction sequence:
(i) complexing with palladium the acetate of formula (5) according to claim 1 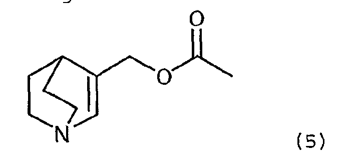
(ii) followed by an allylic amination reaction with phenothiazine leading to dihydromequitazine of formula (6) 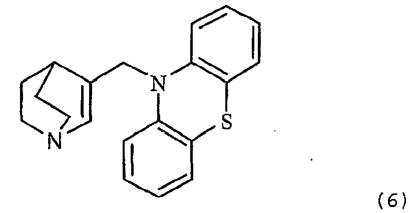
(iii) the latter being subjected to catalytic hydrogenation leading to mequitazine of formula (7)

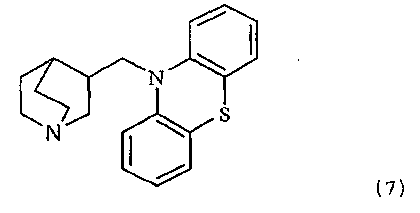

5. Process according to claim 4, **characterised in that** catalytic hydrogenation is carried out under enantiomer-selective conditions.

6. Process for the preparation of the acetate of formula (5) according to claim 1, **characterised in that** it is obtained by allylic substitution of the allylic alcohol of quinuclidine of formula (3)
